# EUROPEAN PATENT SPECIFICATION

(11) **EP 2 590 666 B1**
(45) Date of publication and mention of the grant of the patent: **26.04.2017**
(21) Application number: 11735595.8
(22) Date of filing: 05.07.2011
(51) Int. Cl.: A61K 38/18, A61K 35/14, A61K 35/16, A61K 35/19, A61K 35/28, A61K 9/00, A61K 9/06, A61K 9/127, A61K 31/07, A61K 31/355, A61K 31/375, A61K 47/38, A61P 37/02

(54) **TOPICAL APPLICATION OF ERYTHROPOIETIN FOR USE IN THE TREATMENT OF INJURIES OF THE CORNEA**
TOPISCHE ANWENDUNG VON ERYTHROPOETIN ZUR BEHANDLUNG VON VERLETZUNGEN DER HORNHAUT
APPLICATION TOPIQUE D'ÉRYTHROPOÏÉTINE POUR LE TRAITEMENT DE LÉSIONS DE LA CORNÉE

(30) Priority: 06.07.2010 EP 10006960
(43) Date of publication of application: 15.05.2013
(73) Proprietor: Bader, Augustinus, 04668 Parthenstein / OT Klinga (DE)
(72) Inventor: Bader, Augustinus, 04668 Parthenstein / OT Klinga (DE)
(74) Representative: Benz, Jürgen
(86) International application number: PCT/EP2011/003328
(87) International publication number: WO 2012/003960

(56) References cited:
- EP-A1- 1 481 668
- WO-A1-91/11200
- WO-A1-2005/070451
- WO-A1-2007/128222
- WO-A1-2010/031096
- WO-A2-2009/061447
- US-A1- 2002 061 849
- US-A1- 2006 034 799
- US-A1- 2010 098 675
- KING ET AL: "Erythropoietin is both neuroprotective and neuroregenerative following optic nerve transection", EXPERIMENTAL NEUROLOGY, ACADEMIC PRESS, NEW YORK, NY, US, vol. 205, no. 1, 25 April 2007 (2007-04-25), pages 48-55, XP022046621, ISSN: 0014-4886, DOI: DOI:10.1016/J.EXPNEUROL.2007.01.017

## Description

### TECHNICAL AREA OF THE INVENTION:

The invention relates to the use of erythropoietin (EPO), in particular EPO in a pharmaceutical preparation for the improvement of the vision of a patient, in particular in the case mechanical or pathological injuries of the cornea and conditions related thereto.

In particular, the invention is directed to the topical administration and application of EPO, for example, by eye drops or gel-like formulations directly into the eye.

It includes also the simultaneous application of cells from peripheral blood, blood platelets or cells from bone marrow or the eye, as well as the application of specific vitamins and / or antiseptic agents.

### TECHNICAL BACKGROUND OF THE INVENTION

Erythropoietin (EPO) is a glycoprotein hormone which controls the formation of erythrocytes from precursor cells in the bone marrow (erythropoiesis). EPO binds here to its receptor (EPO-R) and / or its tissue protective subunit the beta Cr (common receptor), which is expressed not only in all haemopoietic cells, but also in cells of the eye and neurons. Previously the sites of erythropoietin formation were believed to be restricted to the kidney, more precisely in the endothelial cells of the peritubular capillaries. Relatively small amounts are also synthesized in the liver cells (hepatocytes).

The principal action of EPO thus consists in increasing the number of red blood corpuscles in the blood, which results in increased oxygen uptake.

In recent years, diverse authors have reported that EPO also exerts a non-haemopoietic action and EPO-R is correspondingly also expressed by certain non-haemopoietic cells. Thus, stimulation by EPO of nerve cells, neuronal cells of the brain and endothelial cells is reported, in some cases together with direct expression of the haemopoietic EPO receptor. In other cases, the presence of a further, non-haemopoietic receptor is prognosticated, but this has not yet been proven in the literature. This receptor (beta Cr) is expressed on stem cells of the eye including CD 90 positive cells.

Increasing importance is being accorded, in particular, to the non-haemopoietic action, which has not been known for very long, of erythropoietin (EPO) in connection, for example, with the stimulated formation and regeneration of endothelial and tissue cells, such as connective tissue, muscle tissue, epithelial tissue and nerve tissue.

WO 02/053580 describes the stimulating effect of EPO and preferably specifically modified EPO derivatives on the generation of a considerable diversity of tissue for the treatment numerous medical indications.

WO 2004/001023 describes, inter alia, the use of EPO and TPO for stimulating neovascularisation and tissue regeneration and improving wound healing, for example after operations or injuries.

WO 2005/063965 teaches the use of EPO for the targeted, structurally controlled regeneration of traumatized tissue, in which not only endothelial cell growth is stimulated, but parenchymal regeneration and the formation of wall structures are also promoted, meaning that coordinated three-dimensional growth occurs for the construction of a functioning tissue, organ or parts thereof.

Erythropoietin, and EPO derivatives or also EPO mimetics, thus appear to be highly suitable on systemic use for specifically initiating and controlling neo-formation and regeneration of the affected tissue in the case of injuries to the skin, the mucous membrane, in the case of open skin and flesh wounds or also in the case of skin irritation caused by burns or scalds, and is ultimately able to promote and accelerate healing.

EP 1 779 862 B1 and WO 2005/070450 describe the use of EPO in the regeneration of vessels and tissue with a weekly dose of less than 90 IU/kg of body weight (= BW), including for the area of wound care. Although possible topical application is theoretically mentioned here, systemic application is preferred. It is therefore postulated to administer EPO in the case of systemic application in a sub-polycythemic weekly dose of less than 90 IU (international units) / kg of body weight (BW) instead of 150 - 300 IU / kg of BW, as was hitherto usual for the known EPO applications.

Although some of the prior art documents mention topical application of EPO for the regeneration of tissue, systemic application of the active compound is, however, placed clearly in the foreground since, on the basis of the results, the non-haematoopoietic EPO effect found can be attributed, in the opinion of more recent papers, primarily to the newly discovered stimulation of corresponding endothelial, vascular or CD31-positive precursor cells, which circulate principally with the blood stream, and only secondarily to the growth of parenchymal tissue structures stimulated thereby.

Thus, the topical administration of EPO for repairing and regeneration of tissue is based on mere speculations in most of the prior art documents and only a few of them provide support by strong data. On topical application of EPO, according to expert opinion to date, merely the inadequate distribution and reachability of the said systemically occurring cells or precursor cells by EPO means that only an unsatisfactory effect, or none at all, on tissue regeneration will be observed.

In the case of regeneration of tissues after, for example, burn trauma or scalding or also in the case of ischaemic wounds, it is necessary to achieve rapid defect closure. This can only occur if the formation of the parenchymal components of the skin is also stimulated as quickly as possible. The time-shifted stimulation of one component (CD31), in order then to facilitate the formation of another component (parenchyma), corresponds to the teaching of the authors of WO 2005/070450 and further publications.

WO 2009/083203 finally discloses EPO comprising formulations, especially hydrogels, based on polysaccharides, e.g Hydroxmethylceluloses, which are useful for topical administration of skin wounds generated by mechanical or pathological injuries.

Topical application of a protein such as EPO is still regarded as a certain challenge because of dramatic losses of action to be expected due to proteolysis of EPO due to enzymatic or other processes in the skin or other tissue wound, and therefore bias exist to use EPO effectively for tissue generation in a topical application approach. Furthermore, since EPO is known to promote endothelial processes (see above) wherein blood vessels and their generation play an important role, it seems prima facie not to be obvious to use EPO for the (re)generation of tissue that is not optimally supplied with blood. Therefore, the use of EPO in a topical administration form for treating eye injuries and disorders and eye disorders based on eye injuries was not suggested so far.

### SUMMARY AND DETAILS OF THE INVENTION:

Surprisingly, it has now been found that, on topical application, EPO is effective in the treatment of eye injuries or pathologically induced damage to the cornea of the eye.

In one aspect of the invention, EPO can be successfully used for the treatment of refractory corneal ulceration.

As here further described here, EPO can be successfully used for the treatment of eye disorders correlated with a "dry eye", or with endothelial dysfunction or dystrophies of the eye, which may occur during or after surgery or with diseases that are caused of said endothelial dysfunction or dystrophy. As further described here EPO can be successfully used for preventing or abolishing scars after eye surgery or injuries.

In another aspect, the invention provides liquid or gel formulations comprising EPO, wherein by specific methods or specific ingredients the effect of the drug can be significantly and enhanced. Thus, by using micelle or liposome vehicles for EPO or other drugs, the availability of EPO and co-drugs even in deeper structures of the eye can be strongly enhanced.

In a further aspect of the invention the addition of co-effective drugs or tissue can surprisingly and synergistically enhance the therapeutic effect of EPO in the eye. Thus, the addition of preferably autologous blood platelets, plasma, stem cells, bone marrow cells and the like causes strong enhancement of the therapeutic effect in the eye. Furthermore, co-drugs such as vitamins or antiseptic agents such as polyhexamethylene biguanide ("polyhexanide"; PHMB) increase strongly the effect of EPO in liquid or gel formulations administered to the eye, even if the eye disease is not primarily caused by microorganisms.

Generally, according to the invention, by administering the EPO to the diseased eye, the mostly strong reduction of vision can be improved by administration of EPO for maximum 14 days by 30 - 50%. Therefore, in case where the eye disease has led to a loss of vision by 30 - 50%, full vision of approximately 100% can be restored.

Refractory corneal ulcers are superficial ulcers that heal poorly and tend to recur. They are also known as indolent ulcers. They are believed to be caused by a defect in the basement membrane and a lack of hemidesmosomal attachments. They are recognized by undermined epithelium that surrounds the ulcer and easily peels back. Refractory corneal ulcers are often accompanied by diabetes mellitus or immunodeficiency disorders.

Refractory corneal ulcers can take a long time to heal, sometimes months according to the methods known in the art. In case of progressive or non-healing ulcers, surgical intervention by an ophthalmologist with corneal transplantation may be required to save the eye.

By applying low doses of EPO (5 - 100 IU EPO per single dose/drop , e.g. 25 - 500 IU / 100 µl) in liquid or gel formulations, for example, administered drop-wise, the vision of patients suffering from a diversity of eye diseases as specified above and below can be strongly improved. For example, in cases where the patients had a reduced vision of 70-80% caused by their disease before treatment, 100% of their original vision can be restored after treatment with EPO within one to four weeks.

EPO eye drops as described in this application can surprisingly prevent all symptoms occurring in connection with a dry eye. Such dry eye phenomena, which can be successfully treated with EPO, may occur also in context with the Sjögren's syndrome. Sjögren's syndrome (also known as "Sicca syndrome") is an autoimmune disorder in which immune cells attack and destroy the exocrine glands that produce tears and saliva. EPO may not cure the syndrome but can surprisingly improve, reduce or even abolish the dry eye symptom. The therapeutic, especially vision improving effect of the EPO formulations extends further to the deeper structures of the eye, the retina included.

In a preferred embodiment of the invention the EPO formulations contain one or more vitamins, preferably vitamin C, A, E and Q (coenzyme Q). Surprisingly the addition of these vitamins solely or in combination improve the therapeutic effect of the formulation. Further improvement can be obtained when the vitamins are administered to the formulation in form of a micelle or liposomal structure. The effect is strong, probably to a surprising improved penetration (30 - 50%) of the drugs (vitamins, EPO) into the deeper eye structure. Similar results can be obtained, if EPO is provided in such a micelle structure. A very useful application of the eye EPO treatment according to the invention is the combination of EPO with the above mentioned vitamins A plus C which is effective in the treatment of diabetic retinopathy or keratomycosis and especially xerophthalmia.

The EPO is given to the formulation according to the invention as solution or as lyophilisate. In preferred embodiments, EPO was introduced into micelle or liposomal structures before applying to the liquid or gel formulation. Here again, a better penetration into deeper structures of the eye, including the retina, can be observed according to the invention. The preparation of micelles or liposomes is well known in the art.

The EPO formulations of the invention (liquid and gel formulations) may contain other drugs or compounds effective in the respective therapy. They may, for example, contain antibiotics or pain killing compounds as known in the art.

The EPO formulations of the invention can be used also for eye disorders of the cornea, such as corneal ulcer / corneal abrasion. Especially the loss of the surface epithelial layer of the eye's cornea is an advantageous application of the EPO eye drop or eye gel therapy as disclosed herein.

These EPO formulations according to the invention contain specific antiseptic compounds, preferably polyhexanide (PHMB) in a concentration of 0.001% - 0.004% (w/w). PHMB (polyhexamethylene biguanide) is known in the art as disinfectant used for surgical and non-surgical wound dressings, better healing of chronic wounds, ulcer, burn wounds, etc. Surprisingly according to the invention, combinations of EPO with PHMB show an enhanced healing effect in the eye with respect to the eye diseases mentioned above and below, preferably after eye surgery, which is more than additive compared to the effect of EPO or PHMB alone (20 - 30% increase compared to the sum of the amount of the single drugs). The enhanced healing effect is apparently deduced to the prevention of primary or secondary infections accompanied with said disease or surgery.

In any case, surprisingly, it could be shown, that EPO-PHMB compositions are very advantageous in the treatment of almost all eye diseases and not only to those that are accompanied or caused primarily by microbial or viral infections. Currently there is no explanation for that in place.

Furthermore, the EPO formulations according of the invention can comprise cells which are autologous to the patient to be treated. Such cells support and improve the effectiveness of EPO. Suitable autologous cells according to the invention are blood cells, blood platelets, blood plasma, bone marrow, limbal or stem cells. The effect of these cells administered within the EPO liquid or gel formulation or separately but preferably simultaneously to the eye is not only an improved and / or accelerated healing effect (e.g. better vision) to the eye, but in addition, allows to reduce strongly the amount of EPO down to 0.1 or 0.2 IU / kg body weight per dose.

According to this disclosure, the EPO formulations if administered for a prolonged treatment period of e.g. 6 months or longer can help to reverse the neuropathic effects in context with eye disorders. The treatment is also applicable to retinopathy, retinopathy of prematurity as well as age-related macular degeneration supporting the regeneration of the photosensitive cells in the macula.

According to this disclosure, the EPO gels and liquids are also applicable to treat peripheral retinal degeneration and assist in retinal haemorrhage and bring together separated retinal layers. They assist in healing processes to reduce macula edema and thus improves distorted central vision, due to a reduction in swelling of the macula. In glaucoma topically administered EPO exerts a strong protective role against neuronal cell death. Pressure elevations can be tolerated more than 2-3 times the normal tolerance ischemic times (20 hours and more instead of 6-7 hours). Prolonged EPO treatment to the eye by means of the EPO formulations according to this disclosure supports remodeling and thus supports healing of disorders of vitreous body and globe. This includes floaters - shadow-like shapes which appear singly or together with several others in the field of vision.

In summary and in detail the invention is related to the following issues:
(i) the use of (human, recombinant) erythropoietin (EPO) for the manufacture of a medicament for the topical treatment of traumas or defects or pathological conditions of the eye, and / or respective methods of treatments, preferably in context with endothelial dysfunction or dystrophy, wherein the trauma, defect or pathological condition is related to the cornea of the eye, or wherein the trauma is a mechanical or pathological injury of the cornea.
(ii) the use of EPO as specified in (i), wherein the pathological condition is refractory cornea ulceration.
(iii) the use of EPO as specified in (i), wherein the treatment abolishes scars on the cornea of the eye.
(iv) the use of EPO as specified in (i) to (iii), wherein deeper structures of the eye including the retina are comprised.
(v) the use of EPO as specified in (i) to (iv), wherein the EPO is provided as a liquid formulation in form of drops, a hydrogel or a liposome formulation.
(vi) the use of EPO as specified in (ix), wherein the EPO is provided for said formulation as lyophilisate or within a micelle or liposome structure.
(vii) the use of EPO for the manufacture of a medicament for improving the vision of a patient suffering from an injury or defect of the cornea of the eye or pathological conditions related thereto, wherein 5 to 300 Units EPO, preferably 10 - 250 IU, 20 - 200 IU, 30 - 250 IU per single dose, or 15 to 600 Units EPO, preferably 100 - 500 IU, 200 - 600 IU per day are topically administered to the patient.
(viii) the use of EPO as specified in (vii) for the improvement of the vision of a patient suffering from an injury or defect of the cornea of the eye or pathological conditions related thereto, wherein 20 to 60 Units EPO / kg body weight / week or less are administered to the patient.
(ix) the use of EPO as specified in (vii), wherein EPO is provided in form of aqueous drops, a hydrogel or a liposome formulation.
(x) the use of EPO as specified in (ix), wherein the EPO is provided for said formulation as lyophilisate or within a micelle or liposomal structure.
(xi) the use of EPO as specified in (ix) and (x), preferably but not limited to preventing infections after eye surgery, wherein the formulation comprises in addition an antiseptic agent, such as polyhexanide (PHMB) or polyaminopropyl biguanide (PAPB).
(xii) the use of EPO as specified in (ix) - (xi), wherein the formulation comprises a vitamin selected from the group consisting of vitamin A, vitamin C, vitamin E, and coenzyme Q, or mixtures thereof, preferably vitamin C and / or vitamin A.
(xiii) the use of EPO as specified in (ix) - (xii), wherein the formulation comprises autologous cells selected from the group consisting of blood plasma, blood platelets, bone marrow, or stem cells, preferably blood platelets.
(xiv) the use of EPO as specified in (xiii), wherein the formulation comprises blood platelets and vitamin C and A, wherein the vitamins and EPO are provided in a micelle structure.
(xv) the use of EPO as specified in (xiii), wherein the formulation comprises platelets, vitamin A and C, and PHMB, wherein, optionally, EPO and / or the vitamins are provided in a micelle structure, for the treatment of eye diseases with or without microbial or viral infections.

The effective dose of EPO can vary dependent on different factors and conditions. According to the invention, EPO is administered by using 5 to 200 IU EPO, preferably 100 to 200 IU EPO, as a single dose solved in 1 to 3 drops (each 20 - 50 µl) water or standard buffered NaCl solution or the like, applied 1 to 5 times, preferably 3 times a day,

A preferred form of calculation relates to the area of eye or the volume of the eye rather than the body weight. This allows to avoid overdosage and is a major benefit of the topical application of EPO, its derivatives or mimetic compounds to the eye.

For corneal ulceration a dosage of 10-100 units EPO per dose is applicable and for treatment of deeper areas or sections of the eye EPO is administered by site specific positioning as drops of 50-100 µl containing 10-100 units by a syringe needle advancing across the ocular bulbus up to the proximity of the retina. Deposition of a depot of such drops will result in a diffusion to the injured, inflamed or degenerating area of the eye to stimulate regeneration of tissue cells.

According to the invention, EPO can be used by topical administration for the treatment of infectious corneal ulceration and can support the usual antibiotic therapy, thus promoting a faster curing process.

In a preferred form autologous plasma of the patient is added by mixing with EPO lyophilisate (e.g. 100-200 Units). Plasma is obtained from the patient according to standard methods typically just prior to the intervention by collection of 5-10 ml of peripheral blood. The plasma is obtained by centrifugation within 5-6 minutes. Blood platelets are collected by enriching the plasma using standard high g centrifugation processes. The plasma, or platelet enriched plasma is than mixed with the EPO lyophilisate containing preferable 10-100 units. In a second preferred embodiment a blood, bone marrow, limbal cells, or adipose tissue derived cell concentrate including plasma, platelets is added topically to the eye together with the EPO. The mixing can be with lyophilisate or with a liquid solution of EPO.

The overall concentration in relationship to the body weight is in this manner only 0,1-0.2 up to 1-2 units / kg body weight. This results in a dramatic reduction in the usage of the protein EPO, meaning cost savings and full avoidance of systemic side effects. The risk of developing allergies is also greatly reduced.

The application of human EPO across species barriers such as in horses or dogs and cats is therefore greatly facilitated.

Especially, in all forms of present, persisting or potential risks of infections to the eye due to injuries, parasites, bacterial contamination or reduced immune status of the patient this combination is dramatically superior as compared to EPO alone.

In a further embodiment vitamins such as vitamin C and A can be added, preferably 1 - 10% (w/w), to the drops or eye gel. A very good penetration to the eye is achieved when the vitamins are added in a form of micellic structure using sorbitol for micelle formation. This greatly improves the penetration into the deeper eye structures by 30-50%.

Surprisingly, in a specific preferred embodiment of the invention, EPO can be applied as a micelle cluster to improve penetration in the eye compared to a standard gel / hydrogel, using e.g. a methylcellulose based carrier (for example hydroxymethyl cellulose), which elicits a slow release mechanism with respect to the topical application of the eye. Micelle structures or clusters with EPO can be achieved by standard techniques. Micelle structures of EPO formed with sorbitol or related compounds are preferred. The use of EPO micelle structures or EPO liposomes increase the therapeutic effect on the diseased eye.

The best results in the treatment of traumatized or diseased eyes, in particular in the case of endothelial dysfunction or dystrophies of the eye, including mechanical or pathological injuries of the cornea and conditions related thereto, as well as injuries following eye surgery, can be obtained according to the invention, when EPO is provided in a micelle structure, optionally together with vitamins provided within a micelle structure, to the liquid or gel formulation. A further synergistic effect can be obtained if further polyhexanide (PHMB) is used in said formulations.

The EPO provided for the current invention is usually applied in form of an aqueous saline-bases solution, or alternatively in form of gel-forming, hydrophilic, moderate- and high-viscosity polymers in form of gels or ointments, such as cellulose derivatives, carbomers, fatty alcohols or macrogols (polyethylene glycols) or mixtures thereof, preferably gel-forming polysaccharides, in particular from the group of the cellulose ethers and cellulose esters, as carrier substance or formulation. The active compound here is uniformly distributed in a viscous, swollen, polymerized or gelatinous matrix, preferably polysaccharide matrix, which protects it and from which it is, in addition, released uniformly and slowly to the surface of the eye and cornea respectively, in which it is able to develop its action directly and immediately without significant decomposition.

Cellulose derivatives are preferably employed for the gels or formulations according to the invention. The group of the cellulose derivatives in connection with the present invention includes, in particular, cellulose ethers and cellulose esters and salts thereof. Cellulose ethers used here are, in particular, hydroxyalkylcelluloses, such as, for example, hydroxymethylcellulose, hydroxyethylcellulose, hydroxypropylcellulose or hydroxybutylcellulose, but in particular hydroxymethylcellulose or hydroxyethylcellulose. Cellulose esters used here are, in particular, carboxyalkylcellulose, in particular carboxymethylcellulose, carboxyethylcellulose, carboxypropylcellulose or carboxybutylcellulose, but preferably carboxymethylcellulose or carboxyethylcellulose, where carboxymethylcellulose is the most preferred.

The EPO containing formulations according to the invention may optionally comprise adjuvants known per se for stabilizing the preparation, including suitable preservatives.

If necessary, for example after eye surgery, the EPO formulation according to the invention may comprise at least one proteinase inhibitor, which is intended to inhibit the proteinases which occur to a massive extent in the wound secretion. A particularly suitable proteinase inhibitor in accordance with the invention is aprotinin, which is capable of inhibiting the pro-inflammatory cytokine secretion which occurs in a wound. Surprisingly, it has been found that EPO is particularly effective for wound healing, preferably in context with eye diseases on topical administration in the presence of aprotinin.

The EPO formulation as described herein are preferably administered into the eye by various routes. The simplest route is to drop or press the liquid or gel formulation into the eye by means of a syringe needle or a pipette (intravitreal application). In case of surgery the medicament can be also applied by intracameral administration. The term "intracameral" means according to the invention an injection of the EPO formulation into the anterior chamber of the eye, usually during surgery.

In the following a list of eye surgeries is provided, that is exemplary only and not limited:
laser eye surgery, cataract surgery, glaucoma surgery, canaloplasty, refractive surgery, corneal surgery, vitreo-retinal surgery, eye muscle surgery, oculoplastic surgery (eyelid surgery, orbital surgery), surgery involving the lacrimal apparatus and eye removal. Corneal transplant surgery, is used to remove a cloudy/diseased cornea and replace it with a clear donor cornea.

An important field of this application is also eye muscle surgery to correct strabism. This involves improved healing after myectomy, myotomy, tenectomy, tenotomy, tightening / strengthening procedures. A further application includes oculoplastic surgery, or oculoplastics. This includes reconstructive or rejuvenating operations in the eye area. Support of eyelid surgery includes blepharoplasty (eyelift) to support healing after removal of excessive skin or subcutaneous fat. The support of orbital surgery includes orbital reconstruction healing following implantation of ocular prosthetics (false eyes)

In cornea transplants the healing of the transplant to the recipient is accelerated (cut) by 50 % when the EPO formulations according to the invention are used. In such cases the eye drops are giving during the operation and for a period of 2 weeks, at 2 times per week following the transplant.

In all forms of surgeries that induce damage to the eye the topical application of the EPO eye drops or gels according to the invention supports the specific healing speed and quality.

For the formulations according to the invention, use is preferably made of recombinant (human) erythropoietin, as is commercially available. However, it is also possible to employ in accordance with the invention EPO derivatives which have been developed in order to extend the half-value period of the active compound in the blood or in the serum compared with native EPO. These include, for example, pegylated EPO, EPO with a modified glycosylation pattern (for example Aranesp®), sialylated EPO, or EPO which has been fused to other polypeptides or fragments of immunoglobulins (for example to the Fc part of an antibody) (known, for example, from WO 02/49673 or WO 01/02017). Furthermore, biologically active synthetic EPO peptide mimetics (as known, for example, from WO 96/40749, WO 96/40772, WO 01/38342, WO 01/091780, WO 2004/101611, WO 2004/100997, WO 2004/101600, WO 2004/101606 and WO 2006/050959) can also be employed.

The following examples are intended to describe the invention in greater detail, but without restricting it in any way. In particular, the person skilled in the art will be able, if desired, to generalize the knowledge from these examples with the aid of his general knowledge.

Furthermore, the substances indicated in the examples, including their parameters, properties, physical quantities, data, and the specific methods described, which the person skilled in the art, unless stated otherwise or prevented by sensible or technical / scientific reasons, will also be able to generalize and bring into other connections than indicated in the examples, should merely be regarded as illustrative.

### EXAMPLES

### Example 1:

20.000 IU E P (*NeoRecormon; powder and solvent for the preparation of an injection solution in cartridges, batch designation MH68260 08, PZN 742 914 3, Roche Reg. Ltd., Welwyn Garden City, UK*) were solved in 1.0 ml NaCl (physiological saline). For topical administration preferably into the eye sack one to three drops of this solution were used by means of a 1 ml syringe, thus containing 166 IU EPO per drop. Per single dose between 166 IU and 500 IU EPO were applied to the patient's eye.

Usually 1 to 3 times per day 1, 2 or 3 drops, each drop containing 150 - 170 IU were given, each or each 2^{nd} day, for one week up to three weeks.

For gel-like preparations for topical administration into the eye, EPO is used in the form of the finished medicament. The active compound is converted into a pre-sterilisable preparation under aseptic conditions using the following gel formers:
∘ Hydroxyethylcellulose Ph, Eur. 5.1 (trade name: Hydroxyethylcellulose 250 HX Pharm, batch designation 06E29-N01, Fagron, D-Barsbüttel)
∘ Carmellose-sodium / carboxymethylcellulose Ph. Eur. 5.0 (trade name Tylopur C600, batch designation 516 762 65, Caelo, D-Hilden)
∘ Methylcellulose / hydroxypropylmethylcellulose USP (trade name Metolose 90 SH-100, batch designation 206314, Shin-Etsu, D-Mülheim)
∘ Povidon Ph. Eur. 5.0 (trade name Kollidön 25, batch designation 74-0915, BASF, D-Ludwigshafen)
5 - 50 µl gel-like preparation containing 50 - 450 IU EPO were applied to the diseased eye.

Further suitable gels are described, for example, in WO 2009//083203.

### Example 2:

A patient suffering from refractory corneal ulcer for a 10 years period, treated before without success by standard therapy, obtained a one week administration with a EPO saline solution as described in above example (3 drops per dose, three times a day, each dose corresponded to 500 IU EPO)

The treatment in an intend to treat module showed a 30 % improvement of the vision in the patient after only 3 days of treatment.

By using 5 to 200 IU EPO, preferably 100 to 200 IU EPO, as a single dose solved in 1 to 3 drops (each 20 - 50 µl) of standard NaCl solution applied 1 to 5 times, preferably 3 times a day, the vision of a first patient group suffering from refractory corneal ulcer can be increased up to 30% after at least one week, preferably after two weeks of preferably topical administration, finally administrating 20 to 60 Units EPO / kg body weight (BW). In cases where the corneal ulcer patients had a reduced vision of 70-80% caused by their disease, 100% of their original vision can be restored after treatment with EPO within one to four weeks.

### Example 3:

In another patient suffering from mechanically induced trauma of the eye, especially the cornea, the vision improvement resulted from 80% vision to 100 % within one week undergoing the same protocol.

### Example 4: Plasma based EPO-PHMB eye drops or gels

The advantage of this variant of the EPO-PHMB formulation according to the invention (with or without vitamin A + C) is the enhancement of regenerative power (factor of 2) over EPO alone. This hitherto unknown synergy of EPO and PHMB is further triggered by the synergy between topical EPO and platelet derived growth factors released on site. In a further embodiment further cell concentrates preferably from peripheral blood (mononuclear cells, progenitors, white and red blood cells, stem cells), bone marrow and fat or even dermal tissue derived cells are used together with the EPO-PHMB formulation. For reason of practicability and reduction of invasiveness the combination of blood derived platelet preparation with or without further addition of autologous blood derived cells is by far the most practical approach. A dramatic synergy is observed and triggered doubling the healing time over conventional methods. A still significant further synergistic effect is observed also against the EPO-PHMB formulation (liquid or gel) without the addition of platelets.

10 ml of peripheral blood is withdrawn from the patient after examining the patient and identification of corneal ulcerations or abrasions. Platelets are obtained by centrifugation according to standard methods. The blood is ideally heparinized to prevent clotting following known routine methods. The platelet enriched plasma, with or without additional cell concentrates from the blood, bone marrow or fat / dermal tissue, is inoculated together with the EPO drops and / or a PHMB concentrate. The final concentration of PHMB is again very low as known from the literature (0.001 - 0.004%).

Vitamins such as coenzyme Q, and / or vitamin C, and / or vitamin E, and / or vitamin A are added on a facultative manner to enhance for the regenerative capacity.

It must be noted that one further advantage of this approach of EPO drops / gel preparation is the replacement of a synthetic gel (e.g. methylcellulose) with a more biocompatible matrix such as plasma.

### Reference Example 1

A third patient with Sjörgensen (Sjögren) syndrome had approx. 30 % vision improvement. Moreover, the dry-eye symptom completely disappeared during the 10 days treatment with a gel-like EPO composition (320 IU per dose, 3 times a day) as described above.

### Reference Example 2:

The same approaches as used in above-specified examples were successfully applied in patients after eye surgeries (including cateract), eye injuries (chemical burns included) or suffering from corneal scars, Fuchs dystrophy, bacterial/mycotic/viral eye infections, and macular degeneration.

### Reference Example 3: Application scheme for infected eyes or for preventing infections to the eye following surgery.

A PHMB containing gel (1 ml, 0.001%) is mixed immediately before application with a liquid or dry (lyophilisate) form of EPO (0.2 ml, 100 units). In addition Vitamin A and Vitamin C in a liquid form is added (0.1 ml) This is done in a sterile manner. 100 µl of the resulting gel are added to the eye with a drop dispenser. The process is repeated every 48 h depending on the half-life of the EPO / anaologue, derivative or biomimetic used. In all patients treated with the above-specified EPO formulation no primary or secondary infection occurred after eye surgery, whereas in the control group (no EPO treatment) more than 20% of the patients suffered from infections after a few days after surgery.

### Reference Example 4 :

The same PHMB-EPO Gel was successfully applied to patients suffering from a diversity of infections of eyelids such as:
- bacterial infection of sebaceous glands of eyelashes
- inflammation of eyelids and eyelashes
- parasitic infestation of the eyelid.
- Dermatitis of the eyelid,

In the case the infectious agent is identified the "eye gel" according to the invention can be easily combined with specific drugs known to be effective against the respective infectious agent.

### Reference Example 5: Treatment of Macular degeneration

The so called "dry" form includes loss of photoreceptors. In this case the EPO drops / gels of the invention are combined with vitamin A and C but PHMB is not needed. However other components are preferably added such as vitamin supplements with high doses of antioxidants, lutein and zeaxanthin.

To slow down macular degeneration the treatment must be repeated at 1-2 times per week and used for several months or longer (more than 6 months).

In wet AMD (aged macula degeneration) a regulating role of vascular angiogenesis is a potential target of the EPO gel of the invention that is at least supportive for other more invasive interventions known in the art and assists in the healing process after such interventions. A mild slow-down of wet macular degeneration is possible.

## Claims

1. EPO for use for the improvement of the vision of a patient suffering from an injury or defect of the cornea of the eye or pathological conditions related thereto, wherein 20 to 60 Units EPO / kg body weight / week are topically administered to the patient in form of aqueous drops, a hydrogel or a liposome formulation.

2. EPO for use according to claim 1, wherein the EPO is provided for said formulation as lyophilisate or within a micelle or liposomal structure.

3. EPO for use according to claim 1 or 2, wherein the formulation comprises polyhexanide (PHMB).

4. EPO for use according to any of the claims 1 - 3, wherein the formulation comprises a vitamin selected from the group consisting of vitamin A, vitamin C, vitamin E and coenzyme Q.

5. EPO for use according to claim 4 for the treatment of infections accompanied with eye diseases or eye surgery.

6. EPO for use according to any of the claims 1 - 5, wherein the topical formulation comprises autologous cells selected from the group consisting of blood plasma, blood platelets, bone marrow cells, or stem cells.

7. EPO for use according to claim 6, wherein the formulation comprises blood platelets.

## Patentansprüche

1. EPO zur Verwendung bei der Verbesserung des Sehvermögens eines Patienten, welcher an einer Verletzung oder einem Defekt der Hornhaut des Auges leidet oder an darauf bezogenen pathologischen Zuständen, wobei 20 bis 60 Einheiten EPO / kg Körpergewicht / Woche dem Patienten topisch verabreicht werden in Form von wässrigen Tropfen, eines Hydrogels oder einer liposomalen Formulierung.

2. EPO zur Verwendung nach Anspruch 1, wobei EPO für besagte Formulierung zur Verfügung gestellt wird als Lyophilisat oder innerhalb einer Mizellen- oder Liposomenstruktur.

3. EPO zur Verwendung nach Anspruch 1, oder 2, wobei die Formulierung Polyhexanid (PHMB) umfasst.

4. EPO zur Verwendung nach einem der Ansprüche 1 - 3, wobei die Formulierung ein Vitamin umfasst, ausgewählt aus der Gruppe, bestehend aus Vitamin A, Vitamin C, Vitamin E und Coenzym Q.

5. EPO zur Verwendung nach Anspruch 4 bei der Behandlung von Infektionen, welche Augenkrankheiten oder Augenoperationen begleiten.

6. EPO zur Verwendung nach einem der Ansprüche 1 - 5, wobei die topische Formulierung autologe Zellen umfasst, ausgewählt aus der Gruppe, bestehend aus Blutplasma, Blutblättchen, Knochenmarkszellen oder Stammzellen.

7. EPO zur Verwendung nach Anspruch 6, wobei die Formulierung Blutplättchen umfasst.

## Revendications

1. EPO destinée à une utilisation pour l'amélioration de la vision d'un patient souffrant d'une lésion ou d'un défaut de la cornée de l'oeil ou de conditions pathologiques y étant liées, où de 20 à 60 unités d'EPO/kg de poids corporel/semaine sont administrées par voie topique au patient sous la forme de gouttes aqueuses, d'un hydrogel ou d'une formulation de liposomes.

2. EPO destinée à une utilisation selon la revendication 1, où l'EPO est fournie pour ladite formulation sous forme de lyophilisat ou au sein d'une micelle ou d'une structure liposomale.

3. EPO destinée à une utilisation selon la revendication 1 ou 2, où la formulation comprend du polyhexanide (PHMB).

4. EPO destinée à une utilisation selon l'une quelconque des revendications 1-3, où la formulation comprend une vitamine choisie dans le groupe constitué par la vitamine A, la vitamine C, la vitamine E et le coenzyme Q.

5. EPO destinée à une utilisation selon la revendication 4, pour le traitement d'infections s'accompagnant de maladies de l'oeil ou d'une chirurgie de l'oeil.

6. EPO destinée à une utilisation selon l'une quelconque des revendications 1-5, où la formulation topique comprend des cellules autologues choisies dans le groupe constitué par le plasma sanguin, les plaquettes sanguines, les cellules de moelle osseuse, ou les cellules souches.

7. EPO destinée à une utilisation selon la revendication 6, où la formulation comprend des plaquettes sanguines.
